(19) Europäisches Patentamt / European Patent Office / Office européen des brevets

(11) **EP 4 647 487 A1**

(12) **EUROPEAN PATENT APPLICATION**

(43) Date of publication:
**12.11.2025   Bulletin 2025/46**

(21) Application number: **25168786.9**

(22) Date of filing: **07.04.2025**

(51) International Patent Classification (IPC):
**C12M 3/06** (2006.01)          **C12M 1/36** (2006.01)
**C12M 1/34** (2006.01)          **C12M 1/00** (2006.01)

(52) Cooperative Patent Classification (CPC):
**C12M 23/16; C12M 31/10; C12M 41/00;**
**C12M 41/44; C12M 41/48**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority:   **24.04.2024   JP 2024070821**

(71) Applicant: **Hamamatsu Photonics K.K.**
**Hamamatsu-shi, Shizuoka 435-8558 (JP)**

(72) Inventors:
• **KUMON, Hiroki**
  **Hamamatsu-shi 435-8558 (JP)**
• **HIRONAKA, Kosuke**
  **Hamamatsu-shi 435-8558 (JP)**

(74) Representative: **Boult Wade Tennant LLP**
**Salisbury Square House**
**8 Salisbury Square**
**London EC4Y 8AP (GB)**

(54) **MICROFLUIDIC DEVICE AND FLOW VELOCITY ESTIMATION METHOD**

(57) In a microfluidic device, a channel is connected to a first container, a culture solution flows through the channel, and a biological sample is disposed in the culture solution. The controller moves the culture solution in the channel to change a liquid amount of the culture solution in the first container. A first light source irradiates the culture solution with a first light passing through a liquid surface of the culture solution. A first light detector detects a first light intensity that is an intensity of the first light that has passed through the culture solution in the first container at a first timing, and a second light intensity that is an intensity of the first light that has passed through the culture solution in the first container at a second timing.

**Fig.1**

EP 4 647 487 A1

**Description**

CROSS REFERENCE

Priority is claimed on Japanese Patent Application No. 2024-070821, filed on April 24, 2024, the entire content of which is incorporated herein by reference.

TECHNICAL FIELD

The present disclosure relates to a microfluidic device and a flow velocity estimation method.

BACKGROUND

Non-patent Literature 1 (Van Duinen, V. et al., "Perfused 3D angiogenic sprouting in a high -throughput in vitro platform", Angiogenesis, Vol.22, pp.157-165 (2019)) and Non-patent Literature 2 (Shinohara Marie et al., "Coculture with hiPS-derived intestinal cells enhanced human hepatocyte functions in a pneumatic-pressure-driven two-organ microphysiological system", Scientific reports, 11.1, 5437 (2021)) disclose a microphysiological system (MPS).

SUMMARY

In recent years, an MPS has been used in cell culture, drug testing, or the like. In a typical configuration example of the MPS, two containers that contain a culture solution are connected by a microchannel. A cell that is a culture target or a test target is disposed in the microchannel. By moving the culture solution from one container to the other container through the microchannel at a constant velocity, the cell can be placed in a constant flow of the culture solution.

Examples of the method for moving the culture solution include using a pump, tilting the container and the microchannel, and the like. In any method, it is necessary to continuously flow the culture solution at a desired flow velocity. Clogging or the like occurs in the microchannel due to some factor, and the culture solution cannot flow at the desired flow velocity, which is a risk, so that it is desirable to continuously monitor the flow velocity of the culture solution. However, since the width of the microchannel of the MPS is as extremely narrow as, for example, 1 mm or less, it is difficult to measure the flow velocity of the culture solution using the conventional flow velocity measurement method.

An object of the present disclosure is to provide a microfluidic device and a flow velocity estimation method capable of easily measuring a flow velocity of a culture solution in a channel that is extremely narrow.

A microfluidic device according to one embodiment of the present disclosure includes a first container, a channel, a controller, a first light source, a first light detector, and an arithmetic processor. The first container is configured to contain a culture solution. One end of the channel is connected to the first container, the culture solution flows through the channel, and a biological sample is disposed in the culture solution in the channel. The controller is configured to move the culture solution in the channel to change a liquid amount of the culture solution in the first container. The first light source is configured to irradiate the culture solution in the first container with a first light passing through a liquid surface of the culture solution. The first light detector is configured to detect a first light intensity that is an intensity of the first light that has passed through the culture solution in the first container at a first timing, and a second light intensity that is an intensity of the first light that has passed through the culture solution in the first container at a second timing different from the first timing. The arithmetic processor is configured to estimate an amount of temporal change between a first optical path length that is an optical path length of the first light in the culture solution of the first container at the first timing and a second optical path length that is an optical path length of the first light in the culture solution of the first container at the second timing, based on an optical density of the culture solution in the first container obtained based on the first light intensity and an optical density of the culture solution in the first container obtained based on the second light intensity. The arithmetic processor is configured to estimate a flow velocity of the culture solution in the channel based on the amount of temporal change.

A flow velocity estimation method according to one embodiment of the present disclosure is a flow velocity estimation method for a microfluidic device. The microfluidic device includes a first container configured to contain a culture solution, and a channel of which one end is connected to the first container, through which the culture solution flows, and in which a biological sample is disposed in the culture solution. The flow velocity estimation method includes starting; detecting a first light intensity; detecting a second light intensity; and estimating. In the starting, an operation of moving the culture solution in the channel to change a liquid amount of the culture solution in the first container is started. In the detecting the first light intensity, the culture solution in the first container is irradiated with a first light passing through a liquid surface of the culture solution, and a first light intensity that is an intensity of the first light that has passed through the culture solution in the first container is detected at a first timing. In the detecting the second light intensity, the culture solution in the first container is irradiated with the first light, and a second light intensity that is an intensity of the first light that has passed through the culture solution in the first container is detected at a second timing different from the first timing. In the estimating, an amount of temporal change between a first optical path length that is an optical path length of the first light in the culture solution of the first container at the first timing and a second optical path length that is an optical path length of the first light in the culture solution of the first container at the second timing is estimated based on an optical density of the culture solution in the first container obtained based on the first light intensity and an optical density of the culture solution in the first container obtained based on the second light intensity. In the estimating, a flow velocity of the culture solution in the channel is estimated based on the amount of temporal change.

According to the present disclosure, it is possible to provide the microfluidic device and the flow velocity estimation method capable of easily measuring the flow velocity of the culture solution in the channel that is extremely narrow.

BRIEF DESCRIPTION OF THE DRAWINGS

FIG. 1 is a perspective view schematically showing a microfluidic device according to one embodiment of the present disclosure.
FIG. 2 is a view for describing a method for calculating an optical density of a culture solution in a first container.
FIG. 3 is a view schematically showing a configuration example of hardware of an arithmetic processor.
FIG. 4 is a view showing a state where an optical axis of a first light source is inclined with respect to a liquid surface of the culture solution in the first container.
FIG. 5 is a graph showing a relationship between a reflectance and an angle at the liquid surface of the culture solution.
FIG. 6 is a flowchart showing a flow velocity estimation method according to one embodiment.
FIGS. 7A to 7C are views showing specific configuration examples of a peripheral structure of the first container and a second container.
FIGS. 8A to 8C are views showing specific configuration examples of the peripheral structure of the first container and the second container.
FIGS. 9A to 9C are views showing specific configuration examples of a peripheral structure of the first container and the second container.
FIGS. 10A to 10C are views showing specific configuration examples of a peripheral structure of the first container and the second container.

DETAILED DESCRIPTION

Specific examples of the present embodiment will be described with reference to the drawings as necessary. The present invention is not limited to these examples but is defined by the claims, and it is intended that the present invention includes all modifications within the concept and scope equivalent to the claims. In the following description, the same elements in the description of the drawings are denoted by the same reference signs, and duplicate descriptions will not be repeated.

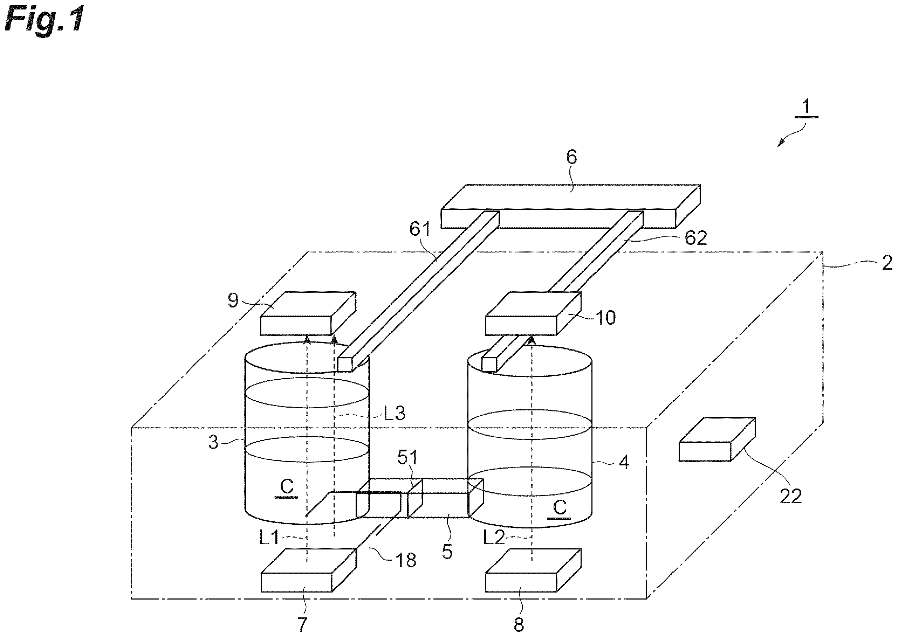

FIG. 1 is a perspective view schematically showing a microfluidic device 1 according to one embodiment of the present disclosure. The microfluidic device 1 includes a first container 3, a second container 4, a channel 5, a controller 6, a first light source 7, a second light source 8, a first light receiver 9 (first light detector), a second light receiver 10 (second light detector), and an arithmetic processor 22.

The first container 3 and the second container 4 contain a culture solution C. The first container 3 and the second container 4 are, for example, tubular containers having a bottom surface, and upper portions thereof are open. The cross-sectional area of each of the first container 3 and the second container 4 in a horizontal plane is constant in a depth direction. The first container 3 and the second container 4 are disposed side by side in a horizontal direction. In one example, the volume of the second container 4 is equal to the volume of the first container 3. The volume of the first container 3 and the second container 4 is, for example, in a range of 50 $\mu$l to 1000 $\mu$l.

The channel 5 connects a bottom portion of the first container 3 to a bottom portion of the second container 4. Namely, one end of the channel 5 is connected to the bottom portion of the first container 3, and the other end of the channel 5 is connected to the bottom portion of the second container 4. The culture solution C flows from the first container 3 to the second container 4 through the channel 5. In the channel 5, a biological sample that is a test target or a culture target is disposed in the culture solution C. The biological sample may be, for example, a cell, a tissue, a cell aggregate, or the like. The channel 5 is, for example, a microchannel. The microchannel is a channel having a cross-sectional dimension less than 1 mm and larger than 1 $\mu$m. For example, when the shape of a cross section 51 of the channel 5, which is a cross section perpendicular to a flow direction of the culture solution C, is a square, a length of one side of the square is larger than 1 $\mu$m and less than 1 mm. In other words, the area of the cross section 51 is larger than 1 $\mu$m$^2$ and less than 1 mm$^2$. The shape of the cross section 51 is not limited to a square, and may be various shapes.

The controller 6 moves the culture solution C in the channel 5 to change the liquid amount of the culture solution C in the first container 3 and the second container 4. The controller 6 moves the culture solution C between the first container 3 and the second container 4 through the channel 5. Therefore, the amount of increase of the culture solution C in the second container 4 is equal to the amount of reduction of the culture solution C in the first container 3. The controller 6 includes, for example, a pressure pump provided in one or both of the first container 3 and the second container 4 to change the air pressure on the liquid surface of the culture solution C. In this case, an air feed pipe 61 extends from the controller 6 to an upper portion of the first container 3, and an air intake pipe 62 extends from the controller 6 to an upper portion of the second container 4.

The controller may include an actuator that tilts the first container 3, the second container 4, and the channel 5, instead of the pressure pump described above. In this case, the actuator moves the culture solution C from the first container 3 to the second container 4 through the channel 5 by tilting the first container 3, the second container 4, and the channel 5. Alternatively, the controller may include a flow pump provided in the channel 5 to move the culture solution C, instead of the pressure pump and the actuator described above. In this case, the culture solution C is moved from the first container 3 to the second container 4 through the channel 5 by the flow pump.

The first light source 7 irradiates the culture solution C in the first container 3 with a first light L1 passing through the liquid surface of the culture solution C. The first light source 7 is, for example, a laser light source, and the first light L1 is, for example, laser light. The second light source 8 irradiates the culture solution C in the second container 4 with a second light L2 passing through the liquid surface of the culture solution C. The second light source 8 is, for example, a laser light source, and the second light L2 is, for example, laser light. The wavelengths of the first light L1 and the second light L2 are included, for example, in a near-infrared range. The wavelength of the first light L1 may be equal to or different from that of the second light L2. The first container 3 and the second container 4 have, for example, a light transmittance of 90% or more at the wavelengths of the first light L1 and the second light L2, respectively. In one example, the optical axis of each of the first light source 7 and the second light source 8 is perpendicular to the liquid surface of the culture solution C. Alternatively, the optical axis of each of the first light source 7 and the second light source 8 may be inclined with respect to the liquid surface of the culture solution C.

The first light receiver 9 detects a first light intensity and a second light intensity. The first light intensity is an intensity of the first light L1 that has passed through the culture solution C in the first container 3 at a first timing. The second light intensity is an intensity of the first light L1 that passes through the culture solution C in the first container 3 at a second timing different from the first timing. The first timing and the second timing may be instantaneous or may include a certain period (for example, a period required to detect light). In addition, the first light source 7 may perform irradiation with the first light L1 only at the first timing and the second timing and may not perform irradiation with the first light L1 during other periods, or may perform irradiation with the first light L1 in a continuous period including the first timing and the second timing. The first light receiver 9 includes a photodiode, and in one example, includes an organic photodiode. In the illustrated example, the first light source 7 is disposed on a bottom surface side of the first container 3, and the first light receiver 9 is disposed on an upper opening side of the first container 3; however, the disposition of the first light source 7 and the first light receiver 9 may be reversed.

The second light receiver 10 detects a third light intensity and a fourth light intensity. The third light intensity is an intensity of the second light L2 that has passed through the culture solution C in the second container 4 at the first timing. The fourth light intensity is an intensity of the second light L2 that has passed through the culture solution C in the second container 4 at the second timing. The second light source 8 may perform irradiation with the second light L2 only at the first timing and the second timing and may not perform irradiation with the second light L2 during other periods, or may perform irradiation with the second light L2 in a continuous period including the first timing and the second timing. The second light receiver 10 includes a photodiode, and in one example, includes an organic photodiode. In the illustrated example, the second light source 8 is disposed on a bottom surface side of the second container 4, and the second light receiver 10 is disposed on an upper opening side of the second container 4; however, the disposition of the second light source 8 and the second light receiver 10 may be reversed.

The arithmetic processor 22 estimates a first optical path length and a second optical path length. The first optical path length is an optical path length of the first light L1 in the culture solution C of the first container 3 at the first timing. The arithmetic processor 22 estimates the first optical path length based on an optical density of the culture solution C in the first container 3 obtained based on the first light intensity. The second optical path length is an optical path length of the first light L1 in the culture solution C of the first container 3 at the second timing. The arithmetic processor 22 estimates the second optical path length based on an optical density of the culture solution C in the first container 3 obtained based on the second light intensity.

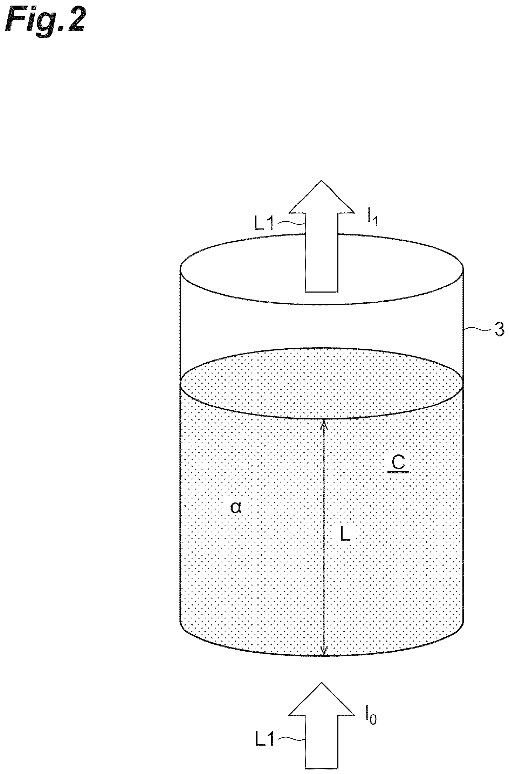

FIG. 2 is a view for describing a method for calculating an optical density of the culture solution C in the first container 3. As shown in FIG. 2, the absorption coefficient of the culture solution C is $\alpha$ (m), the optical path length in the culture solution C is L (m), the light amount of the first light L1 before being incident on the culture solution C is $I_0$, the light amount of the first light L1 after being emitted from the culture solution C is $I_1$, the concentration of the culture solution C is c, and the molar absorption coefficient is $\varepsilon$. In this case, an optical density A is calculated by the following Equation (1). The

light amount $I_0$ is known in advance, and the light amount $I_1$ can be obtained from the first light intensity or the second light intensity.
[Equation 1]

$$A = -log_{10}\left(\frac{I_1}{I_0}\right) \quad \cdots \quad (1)$$
$$= \alpha L = c\varepsilon L$$

The optical density of the culture solution C in the first container 3 at the first timing is $A_{1\_ti}$, the optical density of the culture solution C in the first container 3 at the second timing is $A_{1\_t(i+1)}$, the optical path length (first optical path length) in the culture solution C of the first container 3 at the first timing is $L_{1\_ti}$ (m), and the optical path length (second optical path length) in the culture solution C of the first container 3 at the second timing is $L_{1\_t(i+1)}$ (m). In this case, the following relationship (2) is established between the optical density $A_{1\_ti}$ and the first optical path length $L_{1\_ti}$, and between the optical density $A_{1\_t(i+1)}$ and the second optical path length $L_{1\_t(i+1)}$.
[Equation 2]

$$A_{1\_ti} = \alpha L_{1\_ti} \qquad A_{1\_t(i+1)} = \alpha L_{1\_t(i+1)} \qquad \cdots \quad (2)$$

Therefore, a difference $\Delta L$ (m) between the first optical path length $L_{1\text{-}ti}$ and the second optical path length $L_{1\_t(i+1)}$, namely, the amount of temporal change in the optical path length between the first timing and the second timing is estimated by the following Equation (3).
[Equation 3]

$$\Delta L = L_{1\_ti} - L_{1\_t(i+1)}$$
$$= \frac{1}{\alpha}(A_{1\_ti} - A_{1\_t(i+1)}) \qquad \cdots \quad (3)$$

The arithmetic processor 22 estimates the flow velocity of the culture solution C in the channel 5 based on the amount of temporal change ($\Delta L$) between the first optical path length $L_{1\_ti}$ and the second optical path length $L_{1\_t(i+1)}$. When a time difference $\Delta t$ between the first timing and the second timing is t(i+1) - ti (seconds), the flow velocity $v_m$ (m/s) of the culture solution C in the channel 5 is calculated by the following Equation (4). Here, $Q_m$ (m³/s) is the flow rate of the channel 5 per unit time, $S_m$ (m²) is the area of the cross section 51, and S is the area of the liquid surface of the first container 3.
[Equation 4]

$$v_m = \frac{Q_m}{S_m} = \frac{S\,\Delta L}{S_m\,\Delta t} \qquad \cdots \quad (4)$$

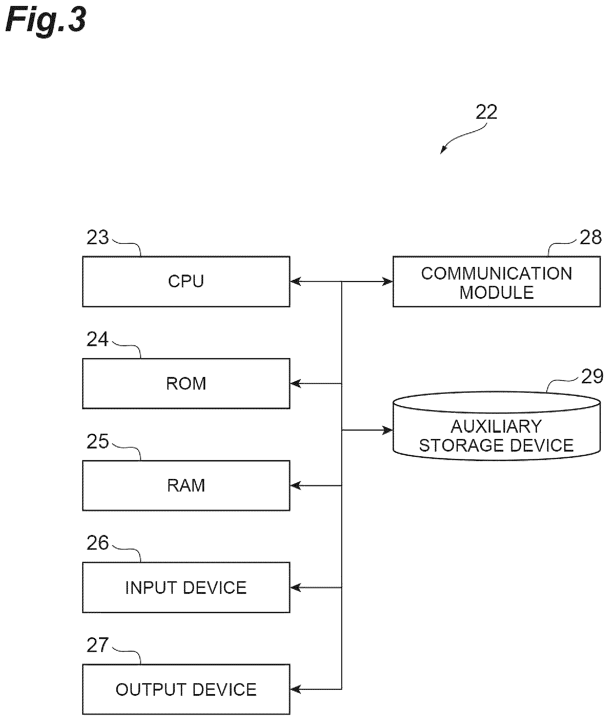

FIG. 3 is a view schematically showing a configuration example of hardware of the arithmetic processor 22. As shown in FIG. 3, the arithmetic processor 22 can be physically configured as a normal computer including a processor (CPU) 23, main storage devices such as a non-volatile memory (ROM) 24 and a volatile memory (RAM) 25, an input device 26 such as a keyboard, a mouse, and a touch screen, an output device 27 such as a display (including a touch screen), a communication module 28 such as a network card for transmitting and receiving data to and from other devices, an auxiliary storage device 29 such as a hard disk, and the like. The input device 26 may receive input regarding the absorption coefficient of the culture solution C or the total liquid amount of the culture solution C from a user.

The processor 23 of the computer can realize the functions of the arithmetic processor 22 described above using a flow velocity estimation program. In other words, the flow velocity estimation program causes the processor 23 of the computer to operate as the arithmetic processor 22. The flow velocity estimation program is stored, for example, in a storage device (storage medium) inside or outside the computer, such as the auxiliary storage device 29. The storage device may be a non-transitory recording medium. Examples of the recording medium include recording media such as a flexible disk, a CD, and a DVD, a recording medium such as a ROM, a semiconductor memory, a cloud server, and the like.

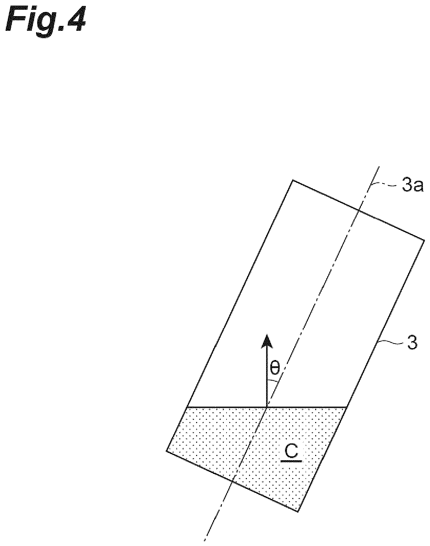

In the above description, a case where the optical axes of the first light source 7 and the second light source 8 are perpendicular to the liquid surface of the culture solution C has been provided as an example. The present invention is not limited thereto, and the optical axis of the first light source 7 may be inclined with respect to the liquid surface of the culture solution C in the first container 3. Similarly, the optical axis of the second light source 8 may be inclined with respect to the liquid surface of the culture solution C in the second container 4. FIG. 4 is a view showing a state where an optical axis 3a of the first light source 7 is inclined with respect to the liquid surface of the culture solution C in the first container 3. Such a mode can occur when a bottom surface of the first container 3, which is normally aligned in the horizontal direction, is inclined with respect to the horizontal direction. For example, in order to move the culture solution C from the first container 3 to the second container 4 through the channel 5, the first container 3, the second container 4, and the channel 5 may be tilted. In the figure, an angle θ formed between a normal vector of the liquid surface of the culture solution C and the optical axis 3a is shown.

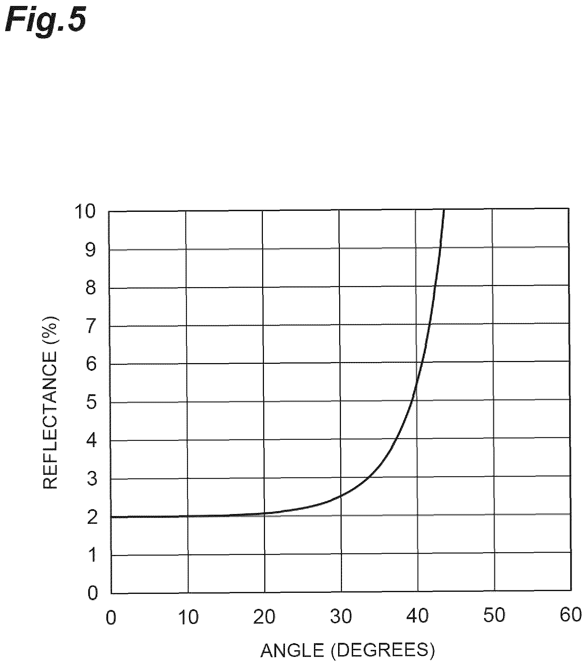

FIG. 5 is a graph showing a relationship between a reflectance and the angle θ at the liquid surface of the culture solution C. In FIG. 5, the horizontal axis represents the angle θ (unit: degree), and the vertical axis represents the reflectance (unit: %). As shown in FIG. 5, when the angle θ is larger than 25 degrees, the reflectance increases rapidly as the angle θ increases. When the reflectance increases, the light intensity detected by the first light receiver 9 decreases, so that it is necessary to perform calculations taking the reflectance into account. On the other hand, when the angle θ is in a range of 25 degrees or less, the reflectance almost does not change compared to when the optical axis is perpendicular to the liquid surface of the culture solution C (namely, angle θ = 0°). When the first container 3 is tilted to move the culture solution C, the angle θ resulting therefrom is a maximum of 25 degrees. Therefore, a decrease in the accuracy of estimation of the flow velocity can be avoided.

Referring again to FIG. 1, the microfluidic device 1 further includes a third light source 18. The third light source 18 irradiates the culture solution C in the first container 3 with a third light L3 having a wavelength different from that of the first light L1. The first light receiver 9 detects a fifth light intensity and a sixth light intensity. The fifth light intensity is an intensity of the third light L3 that has passed through the culture solution C in the first container 3 at a third timing. The sixth light intensity is an intensity of the third light L3 that passes through the culture solution C in the first container 3 at a fourth timing different from the third timing. The first light receiver 9 may detect both the first light L1 and the third light L3 using a single light detection element, or may include a light detection element for detecting the third light L3 separately from a light detection element for detecting the first light L1. The third timing and the fourth timing may be instantaneous or may include a certain period (for example, a period required to detect light). The third timing and the fourth timing may be the same as or different from the first timing and the second timing, respectively. In addition, the third light source 18 may perform irradiation with the third light L3 only at the third timing and the fourth timing and may not perform irradiation with the third light L3 during other periods, or may perform irradiation with the third light L3 in a continuous period including the third timing and the fourth timing. In the illustrated example, the third light source 18 is disposed on the bottom surface side of the first container 3, and the first light receiver 9 is disposed on the upper opening side of the first container 3; however, the disposition of the third light source 18 and the first light receiver 9 may be reversed. The arithmetic processor 22 further estimates a hydrogen ion exponent (pH) of the culture solution C in the first container 3 based on the optical density of the culture solution C in the first container 3 obtained based on the fifth light intensity. In addition, the arithmetic processor 22 may further estimate the amount of change in the hydrogen ion exponent (pH) of the culture solution C in the first container 3 based on the optical density of the culture solution C in the first container 3 obtained based on the fifth light intensity and the optical density of the culture solution C in the first container 3 obtained based on the sixth light intensity. In order to estimate the change in the pH of the culture solution C, for example, a dye such as phenol red that changes color in response to a change in pH may be added to the culture solution C. By measuring the optical density using the third light L3 having a wavelength capable of measuring the change in color caused by the dye, the pH of the culture solution C and a change in pH can be estimated in addition to the flow velocity of the culture solution C. In the method for calculating an optical density, the first light L1 may be replaced with the third light L3 in FIG. 2 described above and the description thereof.

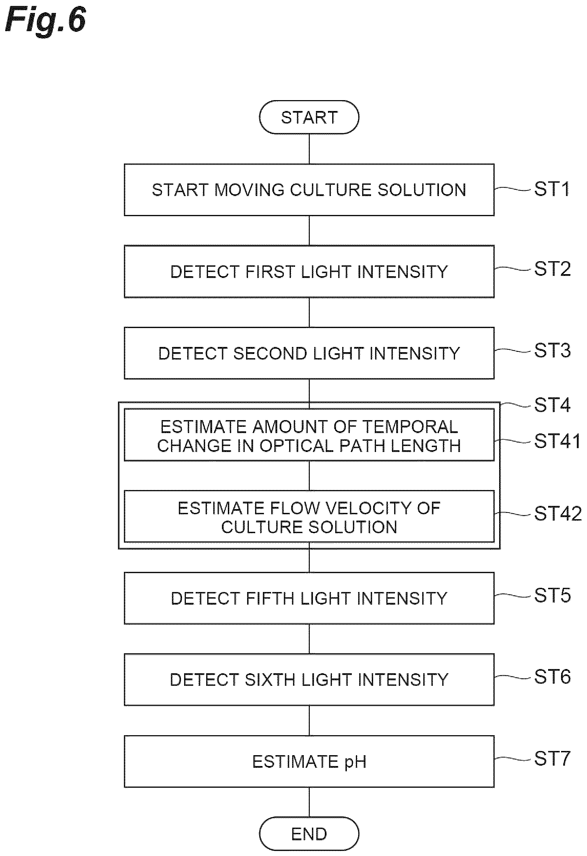

Next, a flow velocity estimation method according to the present embodiment will be described. The flow velocity estimation method of the present embodiment is a flow velocity estimation method for the microfluidic device 1 described above, and can be implemented using, for example, the microfluidic device 1. FIG. 6 is a flowchart showing the flow velocity estimation method according to the present embodiment. As shown in FIG. 6, the flow velocity estimation method includes steps ST1 to ST7.

In step ST1, the controller 6 starts the operation of moving the culture solution C in the channel 5 to change the liquid amount of the culture solution C in the first container 3. At this time, the culture solution C is moved between the first container 3 and the second container 4 through the channel 5. In step ST1, the culture solution C may be moved by tilting the first container 3, the second container 4, and the channel 5, the culture solution C may be moved by changing the air pressure on the liquid surface of the culture solution C in one or both of the first container 3 and the second container 4, or the culture solution C may be moved using the flow pump provided in the channel 5.

In step ST2, at the first timing, the culture solution C in the first container 3 is irradiated with the first light L1 passing through the liquid surface of the culture solution C, and the first light intensity that is the intensity of the first light L1 that has passed through the culture solution C in the first container 3 is detected. In step ST3, at the second timing, the culture solution C in the first container 3 is irradiated with the first light L1, and the second light intensity that is the intensity of the first light L1 that has passed through the culture solution C in the first container 3 is detected. In steps ST2 and ST3, the optical axis of the first light source 7 may be perpendicular to the liquid surface of the culture solution C in the first container 3, or may be inclined with respect thereto.

Step ST4 includes step ST41 and step ST42. In step ST41, the amount of temporal change $\Delta L$ between the first optical path length $L_{1\_ti}$ that is the optical path length of the first light L1 in the culture solution C of the first container 3 at the first timing and the second optical path length $L_{1\_t(i+1)}$ that is the optical path length of the first light L1 in the culture solution C of the first container 3 at the second timing is estimated using Equation (3) described above based on the optical density $A_{1\_ti}$ of the culture solution C in the first container 3 obtained based on the first light intensity, the optical density $A_{1\_t(i+1)}$ of the culture solution C in the first container 3 obtained based on the second light intensity, and the known absorption coefficient $\alpha$ of the culture solution C. In step ST42, the flow velocity $v_m$ of the culture solution C in the channel 5 is estimated using Equation (4) described above based on the amount of temporal change $\Delta L$ between the first optical path length $L_{1\_ti}$ and the second optical path length $L_{1\_t(i+1)}$.

Subsequently, the hydrogen ion exponent (pH) of the culture solution C and the amount of change in pH are estimated. First, in step ST5, the culture solution C in the first container 3 is irradiated with the third light L3, and the fifth light intensity that is the intensity of the third light L3 that has passed through the culture solution C in the first container 3 is detected at the third timing. Next, in step ST6, the culture solution C in the first container 3 is irradiated with the third light L3, and the sixth light intensity that is the intensity of the third light L3 that has passed through the culture solution C in the first container 3 is detected at the fourth timing different from the third timing. Next, in step ST7, the hydrogen ion exponent (pH) of the culture solution C is estimated based on the optical density of the culture solution C in the first container 3 obtained based on the fifth light intensity. In addition, in step ST7, the amount of change in the hydrogen ion exponent (pH) of the culture solution C is estimated based on the optical density of the culture solution C in the first container 3 obtained based on the fifth light intensity and the optical density of the culture solution C in the first container 3 obtained based on the sixth light intensity. In step ST7, only the pH of the culture solution C may be estimated. In this case, step ST6 is unnecessary.

Effects obtained by the microfluidic device 1 and the flow velocity estimation method according to the present embodiment described above will be described. In the microfluidic device 1 of the present embodiment, the arithmetic processor 22 estimates the flow velocity of the culture solution C in the channel 5 based on the amount of temporal change $\Delta L$ in the optical path length of the first light L1 in the culture solution C, the first light L1 passing through the liquid surface of the culture solution C, namely, the amount of temporal change in the position of the liquid surface. The optical path length in the culture solution C can be estimated based on the optical density of the culture solution C obtained based on the light intensity of the first light L1 incident on the first light receiver 9. According to the microfluidic device 1, since it is not necessary to install a device for measuring a flow velocity in the channel 5 that is extremely narrow, the flow velocity of the culture solution C in the channel 5 that is extremely narrow can be easily measured. When the liquid amount of the culture solution C is too large, the distance that the first light L1 passes through the culture solution C becomes longer, so that the attenuation of the first light L1 becomes too large, and the flow velocity of the culture solution C cannot be accurately estimated. The microfluidic device 1 is, for example, a device suitable for measuring a flow velocity in the channel 5 that is extremely narrow, such as a microchannel, in which the liquid amount of the culture solution C is relatively small.

In addition, in the flow velocity estimation method of the present embodiment, the flow velocity of the culture solution C in the channel 5 is estimated based on the amount of temporal change in the optical path length of the first light L1 in the culture solution C, the first light L1 passing through the liquid surface of the culture solution C, namely, the amount of temporal change in the position of the liquid surface. The optical path length in the culture solution C can be estimated based on the optical density of the culture solution C obtained based on the light intensity of the first light L1 incident on the first light receiver 9. According to the flow velocity estimation method, since it is not necessary to install a device for measuring a flow velocity in the channel 5 that is extremely narrow, the flow velocity of the culture solution C in the channel 5 that is extremely narrow can be easily measured. The flow velocity estimation method is also, for example, a method suitable for measuring a flow velocity in the channel 5 that is extremely narrow, such as a microchannel, in which the liquid amount of the culture solution C is relatively small.

As in the present embodiment, the microfluidic device 1 may include the second container 4 that contains the culture solution C and that is connected to the other end of the channel 5. The controller 6 my move the culture solution C between the first container 3 and the second container 4 through the channel 5. Accordingly, the culture solution C can be circulated between the first container 3 and the second container 4, so that the amount of consumption of the culture solution C can be reduced.

As in the present embodiment, the wavelength of the first light L1 may be included in the near-infrared range. The majority of the culture solution C is water, and water absorbs light in the near-infrared range. Therefore, by setting the wavelength of the first light L1 in the near-infrared range, the accuracy of estimation of the first optical path length $L_{1\_ti}$ and the second optical path length $L_{1\_t(i+1)}$ can be improved. In addition, in the microfluidic device 1, the volume of the first container 3 and the second container 4 is as extremely small as, for example, 1 ml or less. In such a small container, light in the near-infrared range is not completely absorbed by the culture solution C. Therefore, the first optical path length $L_{1\_ti}$ and the second optical path length $L_{1\_t(i+1)}$ can be estimated using the first light L1 in the near-infrared range.

As in the present embodiment, the first light receiver 9 may include an organic photodiode. Similarly, in the flow velocity estimation method of the present embodiment, an organic photodiode may be used to detect the first light intensity and the second light intensity. Since the organic photodiode is inexpensive compared to other types of photodiodes, it is easy to discard the organic photodiode together with the first container 3 and the channel 5. Accordingly, unwanted substances (contamination) can be prevented from being mixed into the culture solution C. In addition, the organic photodiode can be designed to receive light of a desired wavelength. Therefore, a wavelength having a high absorption rate in the culture solution C can be selected as the wavelength of the first light L1, so that the accuracy of estimation of the flow velocity of the culture solution C can be improved.

As in the present embodiment, the controller 6 may include an actuator that tilts the first container 3, the second container 4, and the channel 5. In addition, in step ST1, the culture solution C may be moved by tilting the first container 3, the second container 4, and the channel 5. Alternatively, the controller 6 may include a pressure pump provided in one or both of the first container 3 and the second container 4 to change the air pressure on the liquid surface of the culture solution C. In addition, in step ST1, the culture solution C may be moved by changing the air pressure on the liquid surface of the culture solution C in one or both of the first container 3 and the second container 4. Alternatively, the controller 6 may include a flow pump provided in the channel 5 to move the culture solution C. In addition, in step ST1, the culture solution C may be moved using the flow pump provided in the channel 5. For example, the culture solution C in the channel 5 can be moved using any one of these configurations. In addition, in the MPS, it is necessary to continuously flow the culture solution C at a desired flow velocity; however, in these configurations, when clogging occurs in the channel 5 due to some factor, the culture solution C cannot flow at the desired flow velocity. According to the microfluidic device 1 and the flow velocity estimation method of the present embodiment, since it is easy to continuously monitor the flow velocity of the culture solution C, the occurrence of clogging in the channel 5 can be detected at an early stage. Therefore, the microfluidic device 1 and the flow velocity estimation method of the present embodiment are particularly effective for these configurations.

As in the present embodiment, the arithmetic processor 22 may further estimate the pH of the culture solution C in the first container 3 based on the optical density of the culture solution C in the first container 3 obtained based on the fifth light intensity. Similarly, the flow velocity estimation method may include step ST7 of estimating the pH of the culture solution C based on the optical density of the culture solution C in the first container 3 obtained based on the fifth light intensity. Accordingly, the pH of the culture solution C can be estimated in addition to the flow velocity of the culture solution C.

As described above, the optical axis 3a of the first light source 7 may be inclined with respect to the liquid surface of the culture solution C in the first container 3. In this case, even when the first container 3 is tilted to move the culture solution C, the flow velocity of the culture solution C can be suitably estimated.

[First modification example]

The arithmetic processor 22 may estimate the amount of temporal change $\Delta L$ in the optical path length between the first timing and the second timing based on the optical density $A_{1\_ti}$ of the culture solution C in the first container 3 obtained based on the first light intensity and the optical density $A_{1\_t(i+1)}$ of the culture solution C in the first container 3 obtained based on the second light intensity, as well as the optical density of the culture solution C in the second container 4 obtained based on the third light intensity and the optical density of the culture solution C in the second container 4 obtained based on the fourth light intensity.

Specifically, the optical density of the culture solution C in the second container 4 at the first timing is $A_{2\_ti}$, the optical density of the culture solution C in the second container 4 at the second timing is $A_{2\_t(i+1)}$, the optical path length (third optical path length) in the culture solution C of the second container 4 at the first timing is $L_{2\_ti}$ (m), and the optical path length (fourth optical path length) in the culture solution C of the second container 4 at the second timing is $L_{2\_t(i+1)}$ (m). The optical density $A_{2\_ti}$ is obtained by the above Equation (1) based on the third light intensity. The optical density $A_{2\_t(i+1)}$ is obtained by the above Equation (1) based on the fourth light intensity. In this case, the following relationship (5) is established between the optical density $A_{2\_ti}$ and the third optical path length $L_{2\_ti}$, and between the optical density $A_{2\_t(i+1)}$ and the fourth optical path length $L_{2\_t(i+1)}$.

[Equation 5]

$$A_{2\_ti} = \alpha L_{2\_ti} \qquad A_{2\_t(i+1)} = \alpha L_{2\_t(i+1)} \qquad \cdots \ (5)$$

In addition, the following relationship (6) is established among the first optical path length $L_{1\_ti}$, the second optical path length $L_{1\_t(i+1)}$, the third optical path length $L_{2\_ti}$, and the fourth optical path length $L_{2\_t(i+1)}$. Here, $L_{total}$ is the total length of the optical path lengths in the culture solution C.

[Equation 6]

$$L_{1\_ti} + L_{2\_ti} = L_{1\_t(i+1)} + L_{2\_t(i+1)} = L_{total} \qquad \cdots \quad (6)$$

From the above equation, a difference between the first optical path length $L_{1\_ti}$ and the second optical path length $L_{1\_t(i+1)}$, namely, the amount of temporal change $\Delta L$ (m) in the optical path length between the first timing and the second timing is estimated by the following Equation (7).

[Equation 7]

$$\Delta L = L_{1\_ti} - L_{1\_t(i+1)}$$
$$= L_{total}\left(\frac{A_{1\_ti}}{A_{1\_ti} + A_{2\_ti}} - \frac{A_{1\_t(i+1)}}{A_{1\_t(i+1)} + A_{2\_t(i+1)}}\right) \qquad \cdots \quad (7)$$

The arithmetic processor 22 estimates the flow velocity $v_m$ of the culture solution C in the channel 5 using Equation (4) described above based on the amount of temporal change $\Delta L$. In this case, the absorption coefficient $\alpha$ of the culture solution C is not necessary. The arithmetic processor 22 may calculate the total length $L_{total}$ based on the total liquid amount of the culture solution C contained in the first container 3, the second container 4, and the channel 5.

In addition, in step ST2 described above, at the same time that the first light intensity is detected, the culture solution C in the second container 4 may be irradiated with the second light L2 passing through the liquid surface of the culture solution C, and the third light intensity that is the intensity of the second light L2 that has passed through the culture solution C in the second container 4 may be further detected. In addition, in step ST3 described above, at the same time that the second light intensity is detected, the culture solution C in the second container 4 may be irradiated with the second light L2 passing through the liquid surface of the culture solution C, and the fourth light intensity that is the intensity of the second light L2 that has passed through the culture solution C in the second container 4 may be further detected. In this case, in step ST41, the amount of temporal change $\Delta L$ is estimated using Equation (7) described above based on the optical density $A_{1\_ti}$, the optical density $A_{1\_t(i+1)}$, the optical density $A_{2\_ti}$, the optical density $A_{2\_t(i+1)}$, and the total length $L_{total}$. In this case, the absorption coefficient $\alpha$ of the culture solution C is not necessary. In step ST41, the total length $L_{total}$ may be calculated based on the total liquid amount of the culture solution C contained in the first container 3, the second container 4, and the channel 5.

According to the present modification example, even when the absorption coefficient of the culture solution C is unknown, the amount of temporal change $\Delta L$ can be estimated as long as the total length $L_{total}$ of the optical path length of the first light L1 in the culture solution C of the first container 3 and the optical path length of the second light L2 in the culture solution C of the second container 4 is known.

In addition, as described above, in the arithmetic processor 22 and step ST4, the total length $L_{total}$ may be calculated based on the total liquid amount of the culture solution C contained in the first container 3, the second container 4, and the channel 5. For example, with such a configuration, the total length $L_{total}$ can be easily known.

[Specific configuration examples]

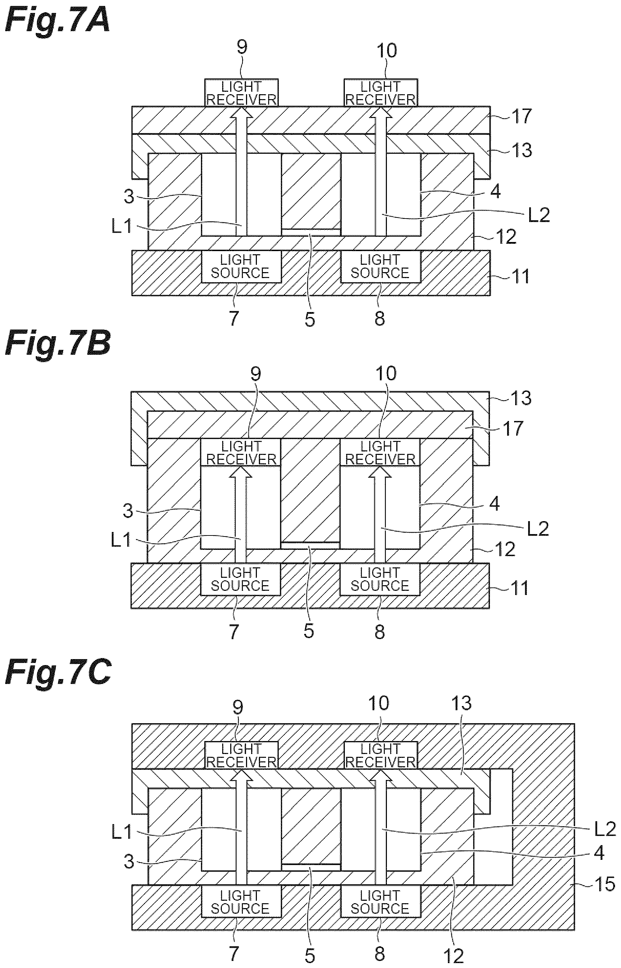

FIGS. 7A to 10C are views showing specific configuration examples of a peripheral structure of the first container 3 and the second container 4. As shown in FIGS. 7A to 10C, the microfluidic device 1 may include a base 11 or a jig 15, a plate 12, and a lid portion 13. The base 11 is an example of a mounting portion in the present disclosure, and the plate 12 is mounted on the base 11. The jig 15 is a member that fixes the lid portion 13 to the plate 12 by sandwiching the plate 12 and the lid portion 13 from above and below. A portion located below the plate 12 is an example of a mounting portion in the present disclosure, and the plate 12 is mounted on the portion. The plate 12 is made of, for example, resin. The first container 3, the second container 4, and the channel 5 are formed in the plate 12. The lid portion 13 is disposed on the plate 12, and closes the upper openings of the first container 3 and the second container 4.

In configurations shown in FIGS. 7A and 7B, the first light source 7 and the second light source 8 are disposed in the base 11. In addition, the first light receiver 9 and the second light receiver 10 are mounted on a substrate 17. The substrate 17 is, for example, a glass substrate or a plastic substrate. In the configuration of FIG. 7A, the substrate 17 is disposed on an upper surface (namely, a surface opposite to a surface facing the first container 3 and the second container 4) of the lid portion 13, and is adhered to the lid portion 13. Furthermore, the first light receiver 9 and the second light receiver 10 are mounted on an upper surface of the substrate 17. In this case, the lid portion 13 and the substrate 17 have a light transmittance of, for example, 90% or more at the wavelengths of the first light L1 and the second light L2. In addition, in the configuration of FIG. 7B, the substrate 17 is disposed on a lower surface (namely, the surface facing the first container 3 and the second container 4) of the lid portion 13, and is adhered to the lid portion 13. Furthermore, the first light receiver 9 and the second light receiver 10 are mounted on a lower surface of the substrate 17. In this case, the lid portion 13 and the substrate 17 may not have light transmissivity, and may block light from the outside.

In a configuration shown in FIG. 7C, the first light source 7 and the second light source 8 are disposed in a portion of the jig 15, which is located below the plate 12. In addition, the first light receiver 9 and the second light receiver 10 are disposed on or above the lid portion 13, and are made in portions of the jig 15, which are located above the lid portion 13. In this case, the lid portion 13 has a light transmittance of, for example, 90% or more at the wavelengths of the first light L1 and the second light L2.

When the microfluidic device 1 has the configuration shown in FIGS. 7A to 7C, in step ST2 and step ST3 of the flow velocity estimation method of the present disclosure, irradiation is performed with the first light L1 and the second light L2 emitted from the first light source 7 and the second light source 8, respectively, that are disposed in the base 11 (or portions of the jig 15, which are located below the plate 12). In addition, the intensities of the first light L1 and the second light L2 are detected by the first light receiver 9 and the second light receiver 10, respectively, that are disposed on or above the lid portion 13.

According to the configurations shown in FIGS. 7A to 7C, the operation of the microfluidic device 1 is simplified, and the burden on a user can be reduced. In addition, the reproducibility of the positions of the optical axes in the first container 3 and the second container 4 is improved. Therefore, a decrease in the accuracy of estimation of the first optical path length $L_{1\_ti}$ and the second optical path length $L_{1\_t(i+1)}$ caused by a variation in the optical axis can be prevented. In addition, since the liquid surface of the culture solution C has a curvature due to a meniscus generated by surface tension, the light diameter of the first light L1 changes when passing through the liquid surface. By disposing the first light receiver 9 and the second light receiver 10 on the lid portion 13, the distance between the liquid surface and both the first light receiver 9 and the second light receiver 10 is reduced. Therefore, the first light L1 can be incident on the first light receiver 9 in a state where the change in the light diameter of the first light L1 is small, and the second light L2 can be incident on the second light receiver 10 in a state where the change in the light diameter of the second light L2 is small.

The present invention is not limited to the above-described examples, and the first light receiver 9 and the second light receiver 10 may be directly made in the lid portion 13. In this case, the substrate 17 is not necessary. In addition, instead of disposing the substrate 17, the first light receiver 9 and the second light receiver 10 may be directly bonded to the lid portion 13 by covalent bonding, ionic bonding, metallic bonding, lamination, or adhesive. The entire surfaces of the first light receiver 9 and the second light receiver 10 may be coated with the adhesive, or only end portions of the first light receiver 9 and the second light receiver 10 may be coated with the adhesive. The first light receiver 9 and the second light receiver 10 may be formed by directly depositing films on the lid portion 13 through sputtering, evaporation, or coating.

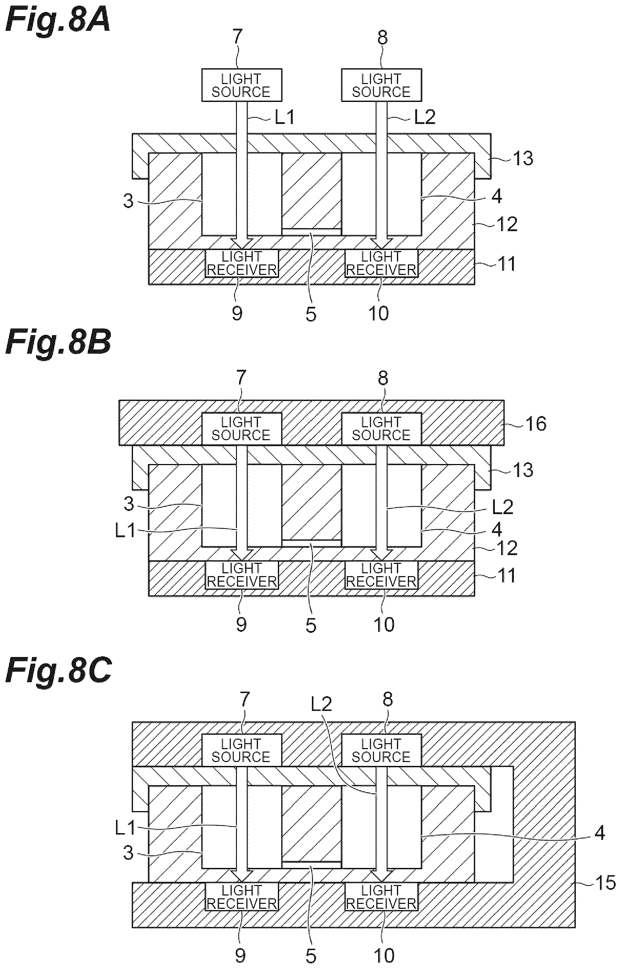

In configurations shown in FIGS. 8A to 8C, the disposition of the first light source 7 and the first light receiver 9 is reversed, and the disposition of the second light source 8 and the second light receiver 10 is reversed. Namely, the first light source 7 and the second light source 8 are disposed on a lid portion 13 side, and the first light receiver 9 and the second light receiver 10 are film-deposited on or affixed to the base 11.

Specifically, in the configuration of FIG. 8A, the first light source 7 and the second light source 8 are disposed above the lid portion 13 and spaced apart from the lid portion 13. The first light L1 and the second light L2 transmit through the lid portion 13, and the culture solution C is irradiated with the first light L1 and the second light L2. In addition, in the configuration of FIG. 8B, the first light source 7 and the second light source 8 are fixed to the upper surface of the lid portion 13 by a jig 16 provided on the lid portion 13. In the configuration of FIG. 8C, the first light source 7 and the second light source 8 are disposed on the lid portion 13, and are disposed in portions of the jig 15, which are located above the lid portion 13.

When the microfluidic device 1 has the configuration shown in FIGS. 8A to 8C, in step ST2 and step ST3 of the flow velocity estimation method of the present disclosure, irradiation is performed with the first light L1 and the second light L2 emitted from the first light source 7 and the second light source 8, respectively, that are disposed on the lid portion 13 or thereabove. In addition, the intensities of the first light L1 and the second light L2 are detected by the first light receiver 9 and the second light receiver 10, respectively, that are disposed below the plate 12.

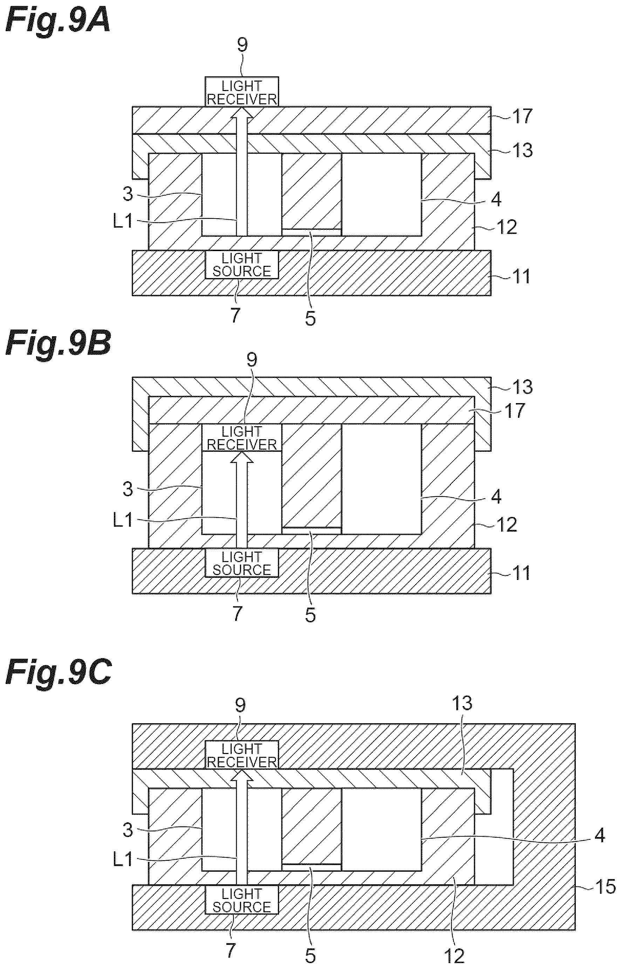

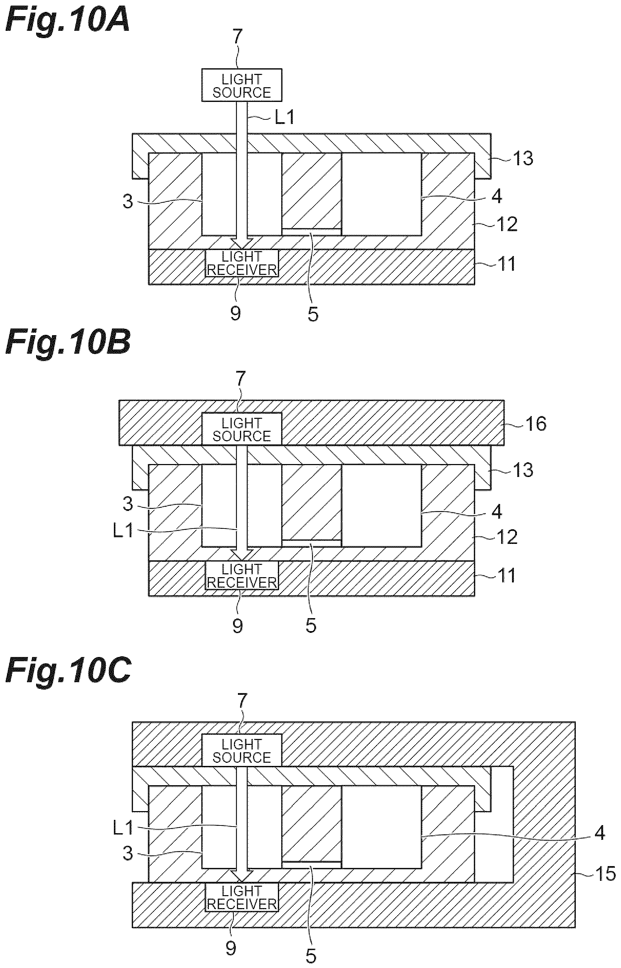

FIGS. 9A to 9C show configurations in which the second light source 8 and the second light receiver 10 are omitted from the configurations shown in FIGS. 7A to 7C, respectively. FIGS. 10A to 10C show configurations in which the second light source 8 and the second light receiver 10 are omitted from the configurations shown in FIGS. 8A to 8C, respectively. When the flow velocity of the culture solution C is estimated using only the first light L1 without using the second light L2 as in the above-described embodiment, the configurations shown in FIGS. 9A to 9C and FIGS. 10A to 10C may be used. Even in this case, the flow velocity of the culture solution C can be suitably estimated by the above Equations (3) and (4).

The microfluidic device and the flow velocity estimation method according to the present disclosure are not limited to the above-described embodiment, and can be modified in various forms. For example, in the above-described embodiment, the first container and the second container are connected to each other by the channel; however, one end of the channel may be connected to the first container, and the other end of the channel may be open without providing the second container. Even in this case, the flow velocity of the culture solution C can be suitably estimated by the above Equations (3) and (4) using only the first light L1.

In addition, in the above-described embodiment, when the absorption coefficient $\alpha$ of the culture solution C is known, the flow velocity of the culture solution C is estimated based on the first optical path length $L_{1\_ti}$ obtained at the first timing and the first optical path length $L_{1\_t(i+1)}$ obtained at the second timing (in other words, using only one of the first light L1 and the second light L2) (refer to Equations (3) and (4)). The present invention is not limited to this mode, and when the total liquid amount of the culture solution C is further known, the flow velocity of the culture solution C may be estimated based on the first optical path length $L_{1\_ti}$ obtained at the first timing and the fourth optical path length $L_{2\_t(i+1)}$ obtained at the second timing (in other words, using one of the first light L1 and the second light L2 at the first timing, and using the other of the first light L1 and the second light L2 at the second timing). In this case, the second optical path length $L_{1\_t(i+1)}$ included in Equation (2) can be calculated based on the known total liquid amount of the culture solution C (in other words, the known total length of the optical path length in the culture solution C of the first container 3 and the optical path length in the culture solution C of the second container 4) and the fourth optical path length $L_{2\_t(i+1)}$.

In the above case, a microfluidic device has the following configuration. Namely, the microfluidic device includes a first container and a second container that contain a culture solution; a channel of which one end is connected to the first container, of which the other end is connected to the second container, through which the culture solution flows, and in which a biological sample is disposed in the culture solution; a controller that moves the culture solution in the channel to change a liquid amount of the culture solution in the first container and the second container; a first light source that irradiates the culture solution in the first container with a first light passing through a liquid surface of the culture solution; a second light source that irradiates the culture solution in the second container with a second light passing through the liquid surface of the culture solution; a first light detector that detects a first light intensity that is an intensity of the first light that has passed through the culture solution in the first container at a first timing; a second light detector that detects a fourth light intensity that is an intensity of the second light that has passed through the culture solution in the second container at a second timing different from the first timing; and an arithmetic processor that estimates an amount of temporal change between an optical path length of the first light in the culture solution of the first container at the first timing and an optical path length of the first light in the culture solution of the first container at the second timing, based on an optical density of the culture solution in the first container obtained based on the first light intensity, an optical density of the culture solution in the second container obtained based on the fourth light intensity, and a total liquid amount of the culture solution, and that estimates a flow velocity of the culture solution in the channel based on the amount of temporal change.

In addition, in the above case, a flow velocity estimation method includes the following steps. Namely, the flow velocity estimation method is a flow velocity estimation method for a microfluidic device, the microfluidic device including a first container and a second container that contain a culture solution, and a channel of which one end is connected to the first container, of which the other end is connected to the second container, through which the culture solution flows, and in which a biological sample is disposed in the culture solution, the flow velocity estimation method including: starting an operation of moving the culture solution in the channel to change a liquid amount of the culture solution in the first container and the second container; irradiating the culture solution in the first container with a first light passing through a liquid surface of the culture solution, and detecting a first light intensity, which is an intensity of the first light that has passed through the culture solution in the first container, at a first timing; irradiating the culture solution in the second container with a second light, and detecting a fourth light intensity, which is an intensity of the second light that has passed through the culture solution in the second container, at a second timing different from the first timing; and estimating an amount of temporal change between a first optical path length that is an optical path length of the first light in the culture solution of the first container at the first timing and a second optical path length that is an optical path length of the first light in the culture solution of the first container at the second timing, based on an optical density of the culture solution in the first container obtained based on the first light intensity and an optical density of the culture solution in the second container obtained based on the fourth light intensity, and estimating a flow velocity of the culture solution in the channel based on the amount of temporal change.

[1] A microfluidic device according to one embodiment of the present disclosure includes a first container, a channel, a controller, a first light source, a first light detector, and an arithmetic processor. The first container is configured to contain a culture solution. One end of the channel is connected to the first container, the culture solution flows through the channel, and a biological sample is disposed in the culture solution in the channel. The controller is configured to move the culture solution in the channel to change a liquid amount of the culture solution in the first container. The first light source is configured to irradiate the culture solution in the first container with a first light passing through a liquid surface of the culture solution. The first light detector is configured to detect a first light intensity that is an intensity of the first light that has passed through the culture solution in the first container at a first timing, and a second light intensity that is an intensity of the first light that has passed through the culture solution in the first container at a second timing different from the first timing. The arithmetic processor is configured to estimate an amount of temporal change between a first optical path length that is an optical path length of the first light in the culture solution of the first container at the first timing and a second optical path length that is an optical path length of the first light in the culture solution of the first container at the second timing, based on an optical density of the culture solution in the first container obtained based on the first light intensity and an optical density of the culture solution in the first container obtained based on the second light intensity. The arithmetic processor is configured to estimate a flow velocity of the culture solution in the channel based on the amount of temporal change.

In the microfluidic device according to the above [1], the arithmetic processor estimates the flow velocity of the culture solution in the channel based on the amount of temporal change in the optical path length of the first light in the culture solution, the first light passing through the liquid surface of the culture solution, namely, an amount of temporal change in the position of the liquid surface. The optical path length in the culture solution can be estimated based on the optical density of the culture solution obtained based on the light intensity of the first light incident on the first light detector. According to the microfluidic device, since it is not necessary to install a device for measuring a flow velocity in the channel that is extremely narrow, the flow velocity of the culture solution in the channel that is extremely narrow can be easily measured. When the liquid amount of the culture solution is too large, the distance that the first light passes through the culture solution becomes longer, so that the attenuation of the first light becomes too large, and the flow velocity of the culture solution cannot be accurately estimated. The microfluidic device is, for example, a device suitable for measuring a flow velocity in the channel that is extremely narrow, such as a microchannel, in which the liquid amount of the culture solution is relatively small.

[2] The microfluidic device according to the above [1] may further include a second container configured to contain the culture solution, and connected to another end of the channel. The controller may be configured to move the culture solution between the first container and the second container through the channel. Accordingly, the culture solution can be circulated between the first container and the second container, and the amount of consumption of the culture solution can be reduced.

[3] The microfluidic device according to the above [2] may further include a second light source and a second light detector. The second light source is configured to irradiate the culture solution in the second container with a second light passing through the liquid surface of the culture solution. The second light detector is configured to detect a third light intensity that is an intensity of the second light that has passed through the culture solution in the second container at the first timing, and a fourth light intensity that is an intensity of the second light that has passed through the culture solution in the second container at the second timing. The arithmetic processor may estimate the amount of temporal change further based on an optical density of the culture solution in the second container obtained based on the third light intensity and an optical density of the culture solution in the second container obtained based on the fourth light intensity. In this case, even when the absorption coefficient of the culture solution is unknown, the amount of temporal change in the optical path length can be estimated as long as the total length of the optical path length of the first light in the culture solution of the first container and the optical path length of the second light in the culture solution of the second container is known.

[4] In the microfluidic device according to the above [3], the arithmetic processor may be configured to estimate the amount of temporal change further based on a total length of the optical path length of the first light in the culture solution of the first container and the optical path length of the second light in the culture solution of the second container.

[5] In the microfluidic device according to the above [4], the arithmetic processor may calculate the total length based on a total liquid amount of the culture solution contained in the first container, the second container, and the channel. For example, with such a configuration, the total length of the optical path length of the first light in the culture solution of the first container and the optical path length of the second light in the culture solution of the second container can be easily known.

[6] In the microfluidic device according to any one of [1] to [5], an optical axis of the first light source may be inclined with respect to the liquid surface of the culture solution in the first container. Accordingly, even when the first container is tilted to move the culture solution, the flow velocity of the culture solution can be suitably estimated.

[7] The microfluidic device according to any one of [1] to [6] may further include a mounting portion on which the first container is mounted; and a lid portion that closes an upper opening of the first container. The first light source may be disposed in the mounting portion, and the first light detector may be disposed on or above the lid portion. In this case, the reproducibility of the position of the optical axis in the first container is improved, so that a decrease in the accuracy of estimation of the first optical path length and the second optical path length can be prevented. In addition, since the liquid surface of the culture solution has a curvature due to a meniscus generated by surface tension, the light diameter of the first light changes when passing through the liquid surface. Since the distance between the first light detector and the liquid surface is reduced by disposing the first light detector on or above the lid portion, the first light can be incident on the first light detector in a state where the change in the light diameter of the first light is small.

[8] In the microfluidic device according to any one of [1] to [7], a wavelength of the first light may be included in a near-infrared range. The majority of the culture solution is water, and water absorbs light in the near-infrared range. Therefore, by setting the wavelength of the first light in the near-infrared range, the accuracy of estimation of the first optical path length and the second optical path length can be improved.

[9] In the microfluidic device according to any one of [1] to [8], the first light detector may include an organic photodiode.

Since the organic photodiode is inexpensive compared to other types of photodiodes, it is easy to discard the organic photodiode together with the first container and the channel. Accordingly, unwanted substances (contamination) can be prevented from being mixed into the culture solution. In addition, the organic photodiode can be designed to receive light of a desired wavelength. Therefore, a wavelength having a high absorption rate in the culture solution can be selected as the wavelength of the first light, so that the accuracy of estimation of the flow velocity of the culture solution can be improved.

[10] In the microfluidic device according to any one of [2] to [9], the controller may include an actuator configured to tilt the first container, the second container, and the channel. [11] Alternatively, the controller may include a pressure pump provided in one or both of the first container and the second container to change an air pressure on the liquid surface of the culture solution. [12] Alternatively, the controller may include a flow pump provided in the channel to move the culture solution. For example, the culture solution in the channel can be moved using any one of the configurations in [10] to [12]. In addition, in these configurations, when clogging occurs in the channel, the culture solution cannot flow at a desired flow velocity, so that the microfluidic device desired above is particularly effective.

[13] The microfluidic device according to any one of [1] to [12] may further include a third light source configured to irradiate the culture solution in the first container with a third light having a wavelength different from a wavelength of the first light. The first light detector may be configured to further detect a fifth light intensity that is an intensity of the third light that has passed through the culture solution in the first container. The arithmetic processor may be configured to further estimate a hydrogen ion exponent (pH) of the culture solution based on an optical density of the culture solution in the first container obtained based on the fifth light intensity. In order to estimate a change in the pH of the culture solution, for example, a dye such as phenol red that changes color in response to a change in pH may be added to the culture solution. By measuring the optical density using the third light having a wavelength capable of measuring the change in color caused by the dye, the pH of the culture solution can be estimated in addition to the flow velocity of the culture solution.

[14] A flow velocity estimation method according to one embodiment of the present disclosure is a flow velocity estimation method for a microfluidic device. The microfluidic device includes a first container configured to contain a culture solution, and a channel of which one end is connected to the first container, through which the culture solution flows, and in which a biological sample is disposed in the culture solution. The flow velocity estimation method includes starting; detecting a first light intensity; detecting a second light intensity; and estimating. In the starting, an operation of moving the culture solution in the channel to change a liquid amount of the culture solution in the first container is started. In the detecting the first light intensity, the culture solution in the first container is irradiated with a first light passing through a liquid surface of the culture solution, and a first light intensity that is an intensity of the first light that has passed through the culture solution in the first container is detected at a first timing. In the detecting the second light intensity, the culture solution in the first container is irradiated with the first light, and a second light intensity that is an intensity of the first light that has passed through the culture solution in the first container is detected at a second timing different from the first timing. In the estimating, an amount of temporal change between a first optical path length that is an optical path length of the first light in the culture solution of the first container at the first timing and a second optical path length that is an optical path length of the first light in the culture solution of the first container at the second timing is estimated based on an optical density of the culture solution in the first container obtained based on the first light intensity and an optical density of the culture solution in the first container obtained based on the second light intensity. In the estimating, a flow velocity of the culture solution in the channel is estimated based on the amount of temporal change.

In the flow velocity estimation method according to the above [14], the flow velocity of the culture solution in the channel is estimated based on the amount of temporal change in the optical path length of the first light in the culture solution, the first light passing through the liquid surface of the culture solution, namely, an amount of temporal change in the position of the liquid surface. The optical path length in the culture solution can be estimated based on the optical density of the culture solution obtained based on the light intensity of the first light incident on the first light detector. According to the flow velocity estimation method, since it is not necessary to install a device for measuring a flow velocity in the channel that is extremely narrow, the flow velocity of the culture solution in the channel that is extremely narrow can be easily measured. When the liquid amount of the culture solution is too large, the distance that the first light passes through the culture solution becomes longer, so that the attenuation of the first light becomes too large, and the flow velocity of the culture solution cannot be accurately estimated. The flow velocity estimation method is, for example, a method suitable for measuring a flow velocity in the channel that is extremely narrow, such as a microchannel, in which the liquid amount of the culture solution is relatively small.

[15] In the flow velocity estimation method according to the above [14], the microfluidic device may further include a second container configured to contain the culture solution, and connected to another end of the channel. In the starting, the culture solution may be moved between the first container and the second container through the channel. In the detecting the first light intensity, the culture solution in the second container may be irradiated with a second light passing through the liquid surface of the culture solution, and a third light intensity that is an intensity of the second light that has passed through the culture solution in the second container may be further detected at the first timing. In the detecting the second light intensity, the culture solution in the second container may be irradiated with the second light, and a fourth light intensity that is an intensity of the second light that has passed through the culture solution in the second container may be further detected at the second timing. In the estimating, the amount of temporal change may be estimated further based on an optical density of the culture solution in the second container obtained based on the third light intensity and an optical density of the culture solution in the second container obtained based on the fourth light intensity. In this case, even when the absorption coefficient of the culture solution is unknown, the amount of temporal change in the optical path length can be estimated as long as the total length of the optical path length of the first light in the culture solution of the first container and the optical path length of the second light in the culture solution of the second container is known.

[16] In the flow velocity estimation method according to the above [15], in the estimating, the amount of temporal change may be estimated further based on a total length of the optical path length of the first light in the culture solution of the first container and the optical path length of the second light in the culture solution of the second container.

[17] In the flow velocity estimation method according to the above [16], in the estimating, the total length may be calculated based on a total liquid amount of the culture solution contained in the first container, the second container, and the channel. For example, with such a method, the total length of the optical path length of the first light in the culture solution of the first container and the optical path length of the second light in the culture solution of the second container can be easily known.

[18] In the flow velocity estimation method according to any one of the above [14] to [17], an optical axis of the first light may be inclined with respect to the liquid surface of the culture solution in the first container. Accordingly, even when the first container is tilted to move the culture solution, the flow velocity of the culture solution can be suitably estimated.

[19] In the flow velocity estimation method according to any one of the above [14] to [18], the microfluidic device may further include a mounting portion on which the first container is mounted, and a lid portion that closes an upper opening of the first container. In the detecting the first light intensity and the detecting the second light intensity, irradiation may be performed with the first light from a light source disposed in the mounting portion, and an intensity of the first light may be detected by a light detector disposed on or above the lid portion. In this case, as described above, the reproducibility of the position of the optical axis in the first container is improved, so that a decrease in the accuracy of estimation of the first optical path length and the second optical path length can be prevented. In addition, since the distance between the light detector and the liquid surface is reduced, the first light can be incident on the light detector in a state where the change in the light diameter of the first light is small.

[20] In the flow velocity estimation method according to any one of the above [14] to [19], a wavelength of the first light may be included in a near-infrared range. In this case, as described above, the accuracy of estimation of the first optical path length and the second optical path length can be improved.

[21] In the flow velocity estimation method according to any one of the above [14] to [20], an organic photodiode may be used to detect the first light intensity and the second light intensity. In this case, as described above, contamination of the culture solution can be prevented. In addition, the accuracy of estimation of the flow velocity of the culture solution can be improved.

[22] In the flow velocity estimation method according to any one of the above [15] to [21], in the starting, the culture solution may be moved by tilting the first container, the second container, and the channel. [23] Alternatively, in the starting, the culture solution may be moved by changing an air pressure on the liquid surface of the culture solution in one or both of the first container and the second container. [24] Alternatively, in the starting, the culture solution may be moved using a flow pump provided in the channel. For example, the culture solution in the channel can be moved using any one of the methods in [22] to [24]. In addition, in these methods, when clogging occurs in the channel, the culture solution cannot flow at a desired flow velocity, so that the flow velocity estimation method described above is particularly effective.

[25] The flow velocity estimation method according to any one of the above [14] to [24] may further include a irradiating the culture solution in the first container with a third light having a wavelength different from a wavelength of the first light, and detecting a fifth light intensity that is an intensity of the third light that has passed through the culture solution in the first container; and a estimating a hydrogen ion exponent (pH) of the culture solution based on an optical density of the culture solution in the first container obtained based on the fifth light intensity. In this case, as described above, the pH of the culture solution can be estimated in addition to the flow velocity of the culture solution.

**Claims**

1. A microfluidic device comprising:

   a first container configured to contain a culture solution;
   a channel of which one end is connected to the first container, through which the culture solution flows, and in which a biological sample is disposed in the culture solution;

a controller configured to move the culture solution in the channel to change a liquid amount of the culture solution in the first container;

a first light source configured to irradiate the culture solution in the first container with a first light passing through a liquid surface of the culture solution;

a first light detector configured to detect a first light intensity that is an intensity of the first light that has passed through the culture solution in the first container at a first timing, and a second light intensity that is an intensity of the first light that has passed through the culture solution in the first container at a second timing different from the first timing; and

an arithmetic processor configured to estimate an amount of temporal change between a first optical path length that is an optical path length of the first light in the culture solution of the first container at the first timing and a second optical path length that is an optical path length of the first light in the culture solution of the first container at the second timing, based on an optical density of the culture solution in the first container obtained based on the first light intensity and an optical density of the culture solution in the first container obtained based on the second light intensity, and configured to estimate a flow velocity of the culture solution in the channel based on the amount of temporal change.

2. The microfluidic device according to claim 1, further comprising:

a second container configured to contain the culture solution and connected to another end of the channel, wherein the controller is configured to move the culture solution between the first container and the second container through the channel.

3. The microfluidic device according to claim 2, further comprising:

a second light source configured to irradiate the culture solution in the second container with a second light passing through the liquid surface of the culture solution; and

a second light detector configured to detect a third light intensity that is an intensity of the second light that has passed through the culture solution in the second container at the first timing, and a fourth light intensity that is an intensity of the second light that has passed through the culture solution in the second container at the second timing,

wherein the arithmetic processor estimates the amount of temporal change further based on an optical density of the culture solution in the second container obtained based on the third light intensity and an optical density of the culture solution in the second container obtained based on the fourth light intensity.

4. The microfluidic device according to claim 3,

wherein the arithmetic processor is configured to estimate the amount of temporal change further based on a total length of the optical path length of the first light in the culture solution of the first container and the optical path length of the second light in the culture solution of the second container.

5. The microfluidic device according to claim 4,

wherein the arithmetic processor calculates the total length based on a total liquid amount of the culture solution contained in the first container, the second container, and the channel.

6. The microfluidic device according to any one of claims 1 to 5, further comprising:

a mounting portion on which the first container is mounted; and

a lid portion that closes an upper opening of the first container,

wherein the first light source is disposed in the mounting portion, and

the first light detector is disposed on or above the lid portion.

7. The microfluidic device according to any one of claims 1 to 6,

wherein a wavelength of the first light is included in a near-infrared range.

8. The microfluidic device according to any one of claims 1 to 7, further comprising:

a third light source configured to irradiate the culture solution in the first container with a third light having a wavelength different from a wavelength of the first light,

wherein the first light detector is configured to further detect a fifth light intensity that is an intensity of the third light that has passed through the culture solution in the first container, and

the arithmetic processor is configured to further estimate a hydrogen ion exponent (pH) of the culture solution based on an optical density of the culture solution in the first container obtained based on the fifth light intensity.

9. A flow velocity estimation method for a microfluidic device, the microfluidic device including a first container configured to contain a culture solution, and a channel of which one end is connected to the first container, through which the culture solution flows, and in which a biological sample is disposed in the culture solution, the flow velocity estimation method comprising:

starting an operation of moving the culture solution in the channel to change a liquid amount of the culture solution in the first container;

irradiating the culture solution in the first container with a first light passing through a liquid surface of the culture solution, and detecting a first light intensity, which is an intensity of the first light that has passed through the culture solution in the first container, at a first timing;

irradiating the culture solution in the first container with the first light, and detecting a second light intensity, which is an intensity of the first light that has passed through the culture solution in the first container, at a second timing different from the first timing; and

estimating an amount of temporal change between a first optical path length that is an optical path length of the first light in the culture solution of the first container at the first timing and a second optical path length that is an optical path length of the first light in the culture solution of the first container at the second timing, based on an optical density of the culture solution in the first container obtained based on the first light intensity and an optical density of the culture solution in the first container obtained based on the second light intensity, and estimating a flow velocity of the culture solution in the channel based on the amount of temporal change.

10. The flow velocity estimation method according to claim 9,

wherein the microfluidic device further includes a second container configured to contain the culture solution and connected to another end of the channel,

in the starting, the culture solution is moved between the first container and the second container through the channel,

in the detecting the first light intensity, the culture solution in the second container is irradiated with a second light passing through the liquid surface of the culture solution, and a third light intensity that is an intensity of the second light that has passed through the culture solution in the second container is further detected at the first timing,

in the detecting the second light intensity, the culture solution in the second container is irradiated with the second light, and a fourth light intensity that is an intensity of the second light that has passed through the culture solution in the second container is further detected at the second timing, and

in the estimating, the amount of temporal change is estimated further based on an optical density of the culture solution in the second container obtained based on the third light intensity and an optical density of the culture solution in the second container obtained based on the fourth light intensity.

11. The flow velocity estimation method according to claim 10,
wherein in the estimating, the amount of temporal change is estimated further based on a total length of the optical path length of the first light in the culture solution of the first container and the optical path length of the second light in the culture solution of the second container.

12. The flow velocity estimation method according to claim 11,
wherein the total length is calculated based on a total liquid amount of the culture solution contained in the first container, the second container, and the channel.

13. The flow velocity estimation method according to any one of claims 9 to 12,

wherein the microfluidic device further includes a mounting portion on which the first container is mounted, and a lid portion that closes an upper opening of the first container, and

in the detecting the first light intensity and the detecting the second light intensity, irradiation is performed with the first light from a light source disposed in the mounting portion, and an intensity of the first light is detected by a light detector disposed on or above the lid portion.

**14.** The flow velocity estimation method according to any one of claims 9 to 13,
wherein a wavelength of the first light is included in a near-infrared range.

**15.** The flow velocity estimation method according to any one of claims 9 to 14, further comprising:

irradiating the culture solution in the first container with a third light having a wavelength different from a wavelength of the first light, and detecting a fifth light intensity that is an intensity of the third light that has passed through the culture solution in the first container; and
estimating a hydrogen ion exponent (pH) of the culture solution based on an optical density of the culture solution in the first container obtained based on the fifth light intensity.

**Fig.1**

EP 4 647 487 A1

# Fig.2

# Fig.3

22

23 CPU

28 COMMUNICATION MODULE

24 ROM

25 RAM

29 AUXILIARY STORAGE DEVICE

26 INPUT DEVICE

27 OUTPUT DEVICE

# Fig.4

# Fig.5

# Fig.6

```
                    ┌─────────────┐
                    │    START    │
                    └──────┬──────┘
                           │
        ┌──────────────────────────────────────┐
        │   START MOVING CULTURE SOLUTION       │──── ST1
        └──────────────────┬───────────────────┘
                           │
        ┌──────────────────────────────────────┐
        │      DETECT FIRST LIGHT INTENSITY     │──── ST2
        └──────────────────┬───────────────────┘
                           │
        ┌──────────────────────────────────────┐
        │     DETECT SECOND LIGHT INTENSITY     │──── ST3
        └──────────────────┬───────────────────┘
                           │                        ST4
        ┌──────────────────────────────────────┐
        │ ┌──────────────────────────────────┐ │
        │ │  ESTIMATE AMOUNT OF TEMPORAL      │ │──── ST41
        │ │  CHANGE IN OPTICAL PATH LENGTH    │ │
        │ └──────────────────────────────────┘ │
        │ ┌──────────────────────────────────┐ │
        │ │    ESTIMATE FLOW VELOCITY OF      │ │──── ST42
        │ │       CULTURE SOLUTION            │ │
        │ └──────────────────────────────────┘ │
        └──────────────────┬───────────────────┘
                           │
        ┌──────────────────────────────────────┐
        │      DETECT FIFTH LIGHT INTENSITY     │──── ST5
        └──────────────────┬───────────────────┘
                           │
        ┌──────────────────────────────────────┐
        │      DETECT SIXTH LIGHT INTENSITY     │──── ST6
        └──────────────────┬───────────────────┘
                           │
        ┌──────────────────────────────────────┐
        │            ESTIMATE pH                │──── ST7
        └──────────────────┬───────────────────┘
                           │
                    ┌─────────────┐
                    │     END     │
                    └─────────────┘
```

**Fig.7A**

**Fig.7B**

**Fig.7C**

EP 4 647 487 A1

**Fig.8A**

**Fig.8B**

**Fig.8C**

25

*Fig.9A*

*Fig.9B*

*Fig.9C*

*Fig.10A*

*Fig.10B*

*Fig.10C*

Europäisches
Patentamt

European
Patent Office

Office européen
des brevets

# EUROPEAN SEARCH REPORT

**Application Number**

EP 25 16 8786

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2018/298324 A1 (TAKEDA KAZUO [JP] ET AL) 18 October 2018 (2018-10-18) * paragraphs [0041], [0082] - [0084]; figure 1 * ----- | 1-15 | INV. C12M3/06 C12M1/36 C12M1/34 C12M1/00 |
| Y | EP 2 645 071 A2 (NIHON KOHDEN CORP [JP]; UNIV TOKYO WOMENS MEDICAL [JP]) 2 October 2013 (2013-10-02) * claim 1; figure 1 * ----- | 1-15 | |
| A | WO 2021/245048 A1 (MERCK PATENT GMBH [DE]) 9 December 2021 (2021-12-09) * claim 1; figure 1 * ----- | 1-15 | |
| A | WO 02/01163 A2 (MICRONICS INC [US]) 3 January 2002 (2002-01-03) * claims 1-13 * ----- | 1-15 | |

|  |
|---|
| **TECHNICAL FIELDS SEARCHED (IPC)** |
| C12M B01L |

The present search report has been drawn up for all claims

| Place of search | Date of completion of the search | Examiner |
|---|---|---|
| Berlin | 16 September 2025 | Jones, Laura |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 25 16 8786

This annex lists the patent family members relating to the patent documents cited in the above-mentioned European search report.
The members are as contained in the European Patent Office EDP file on
The European Patent Office is in no way liable for these particulars which are merely given for the purpose of information.

16-09-2025

| Patent document cited in search report | | | Publication date | Patent family member(s) | | | Publication date |
|---|---|---|---|---|---|---|---|
| US 2018298324 | A1 | | 18-10-2018 | CN | 107532990 | A | 02-01-2018 |
| | | | | JP | 6962563 | B2 | 05-11-2021 |
| | | | | JP | 7283798 | B2 | 30-05-2023 |
| | | | | JP | 2022025077 | A | 09-02-2022 |
| | | | | JP | 2023104940 | A | 28-07-2023 |
| | | | | JP | WO2016182034 | A1 | 29-03-2018 |
| | | | | US | 2018298324 | A1 | 18-10-2018 |
| | | | | US | 2023383240 | A1 | 30-11-2023 |
| | | | | WO | 2016182034 | A1 | 17-11-2016 |
| EP 2645071 | A2 | | 02-10-2013 | EP | 2645071 | A2 | 02-10-2013 |
| | | | | JP | 5869937 | B2 | 24-02-2016 |
| | | | | JP | 2013202031 | A | 07-10-2013 |
| | | | | US | 2013255374 | A1 | 03-10-2013 |
| WO 2021245048 | A1 | | 09-12-2021 | CN | 115666752 | A | 31-01-2023 |
| | | | | EP | 4161671 | A1 | 12-04-2023 |
| | | | | US | 2023168518 | A1 | 01-06-2023 |
| | | | | WO | 2021245048 | A1 | 09-12-2021 |
| WO 0201163 | A2 | | 03-01-2002 | US | 2001054702 | A1 | 27-12-2001 |
| | | | | US | 2002008032 | A1 | 24-01-2002 |
| | | | | US | 2002015959 | A1 | 07-02-2002 |
| | | | | US | 2003197139 | A1 | 23-10-2003 |
| | | | | WO | 0201081 | A2 | 03-01-2002 |
| | | | | WO | 0201163 | A2 | 03-01-2002 |
| | | | | WO | 0201184 | A1 | 03-01-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2024070821 A

**Non-patent literature cited in the description**

- **VAN DUINEN, V et al.** Perfused 3D angiogenic sprouting in a high -throughput in vitro platform. *Angiogenesis*, 2019, vol. 22, 157-165

- **SHINOHARA MARIE et al.** Coculture with hiPS-derived intestinal cells enhanced human hepatocyte functions in a pneumatic-pressure-driven two-organ microphysiological system. *Scientific reports*, 2021, vol. 11 (1), 5437